# EUROPEAN PATENT SPECIFICATION

(11) **EP 1 562 920 B1**
(45) Date of publication and mention of the grant of the patent: **28.06.2006**
(21) Application number: 03770201.6
(22) Date of filing: 06.11.2003
(51) Int. Cl.: C07D 277/22, C07D 277/24, C07D 277/26, C07D 277/28, C07D 417/12, A61K 31/426, A61P 29/00, A61P 19/02, A61P 9/00, A61P 25/06, A61P 25/08, A61P 25/18, A61P 35/00, A61P 37/08, A61P 1/00, A61P 31/12

(54) **ANTIINFLAMMATORY 3-ARYLTHIO-3-THIAZOLYL-ALKYLAMINES**
ENTZÜNDUNGSHEMMENDE 3-ARYLTHIO-3-THIAZOLYLALKYLAMINE
3-ARYLTHIO-3-THIAZOLYL-ALKYLAMINES ANTI-INFLAMMATOIRES

(30) Priority: 07.11.2002 SE 0203304
(43) Date of publication of application: 17.08.2005
(73) Proprietor: AstraZeneca AB, 151 85 Södertälje (SE)
(72) Inventor: STONEHOUSE, Jeffrey, AstraZeneca R & D Charnwood, Loughborough, Leicestershire LE11 5RH (GB)
(86) International application number: PCT/SE2003/001712
(87) International publication number: WO 2004/041794

(56) References cited:
- WO-A1-01/62704
- WO-A1-01/62713
- WO-A1-01/62714
- WO-A1-01/62721
- WO-A2-02/090332

## Description

### Field of the Invention

The present invention relates to novel arylthiazolylthioalkylamine derivatives, processes for their preparation, compositions containing them and their use in therapy.

### Background of the Invention

Nitric oxide is produced in mammalian cells from L-arginine by the action of specific nitric oxide synthases (NOSs). These enzymes fall into two distinct classes - constitutive NOS (cNOS) and inducible NOS (iNOS). At the present time, two constitutive NOSs and one inducible NOS have been identified. Of the constitutive NOSs, an endothelial enzyme (ecNOS) is involved with smooth muscle relaxation and the regulation of blood pressure and blood flow, whereas the neuronal enzyme (ncNOS) serves as a neurotransmitter and appears to be involved in the regulation of various biological functions such as cerebral ischaemia. Inducible NOS has been particularly implicated in the pathogenesis of inflammatory diseases. Regulation of these enzymes should therefore offer considerable potential in the treatment of a wide variety of disease states (J. E. Macdonald, *Ann*. *Rep. Med. Chem*., 1996, **31,** 221 - 230).

Considerable effort has been expended in efforts to identify compounds that act as specific inhibitors of one or more isoforms of the enzyme nitric oxide synthase. The use of such compounds in therapy has also been widely claimed.

Patent application WO 02/090332 discloses certain arylheteroalkylamine derivatives that are useful as NOS inhibitors. It has now been found that certain compounds that are within the generic scope of this application, but which are not specifically exemplified therein, possess surprisingly advantageous properties. These compounds are the subject of the present application.

### Disclosure of the invention

According to the present invention, there is provided a compound of formula (I) 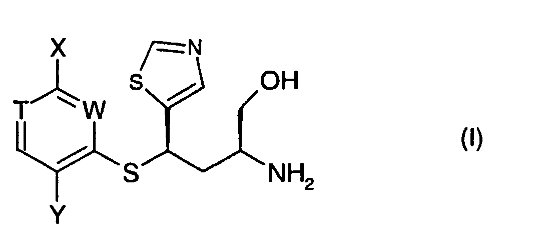 wherein:
T and W independently represent CR¹ or N; and when more than one R¹ group is present, each may be selected independently;
X and R¹ independently represent H, C 1 to 4 alkyl, C 1 to 4 alkoxy, halogen, CN, C≡CH, NO₂, CHO, COCH₃ or NHCHO; said alkyl or alkoxy group being optionally further substituted by one or more fluorine atoms;
Y represents C1 to 4 alkyl, C1 to 4 alkoxy, halogen, CN, C≡CH, NO₂, CHO, COCH₃ or NHCHO; said alkyl or alkoxy group being optionally further substituted by one or more fluorine atoms;
or a pharmaceutically acceptable salt thereof.

In one embodiment, Y represents CN or halogen.

In one embodiment, X and R¹ independently represent H, halogen or CF₃.

The compounds of formula (I) and their pharmaceutically acceptable salts have the advantage that they are inhibitors of the enzyme nitric oxide synthase (NOS). In particular, the compounds of formula (I) and their pharmaceutically acceptable salts have the advantage that they are inhibitors of the inducible isoform of the enzyme nitric oxide synthase (iNOS).

The invention further provides a process for the preparation of compounds of formula (I) or a pharmaceutically acceptable salt, enantiomer or racemate thereof.

According to the invention there is also provided a compound of formula (I), or a pharmaceutically acceptable salt thereof, for use as a medicament.

Another aspect of the invention provides the use of a compound of formula (I) or a pharmaceutically acceptable salt thereof, in the manufacture of a medicament, for the treatment or prophylaxis of diseases or conditions in which inhibition of nitric oxide synthase activity is beneficial.

A more particular aspect of the invention provides the use of a compound of formula (I) or a pharmaceutically acceptable salt thereof, in the manufacture of a medicament, for the treatment or prophylaxis of inflammatory disease.

According to the invention, there is also provided a method of treating, or reducing the risk of, diseases or conditions in which inhibition of nitric oxide synthase activity is beneficial which comprises administering to a person suffering from or at risk of, said disease or condition, a therapeutically effective amount of a compound of formula (I) or a pharmaceutically acceptable salt thereof.

More particularly, there is also provided a method of treating, or reducing the risk of, inflammatory disease in a person suffering from or at risk of, said disease, wherein the method comprises administering to the person a therapeutically effective amount of a compound of formula (I) or a pharmaceutically acceptable salt thereof.

The compounds of the present invention may also be used advantageously in combination with a second pharmaceutically active substance; particularly in combination with a cyclooxygenase inhibitor; more particularly in combination with a selective inhibitor of the inducible isoform of cyclooxygenase (COX-2). Thus, in a further aspect of the invention there is provided the use of a compound of formula (I) or a pharmaceutically acceptable salt thereof, in combination with a COX-2 inhibitor for the treatment of inflammation, inflammatory disease and inflammatory related disorders. And there is also provided a method of treating, or reducing the risk of, inflammation, inflammatory disease and inflammatory related disorders in a person suffering from or at risk of, said disease or condition, wherein the method comprises administering to the person a therapeutically effective amount of a compound of formula (I) or a pharmaceutically acceptable salt thereof in combination with a COX-2 inhibitor.

Particular compounds of the invention include:
2-[[(1*R*,3*S*)-3-amino-4-hydroxy-1-(5-thiazolyl)butyl]thio]-5-chloro-3-pyridinecarbonitrile;
2-[[(1*R*,3*S*)-3-amino-4-hydroxy-1-(5-thiazolyl)butyl]thio]-4-chloro-benzonitrile;
(2*S*,4*R*)-2-amino-4-[[2-chloro-5-(trifluoromethyl)phenyl]thio]-5-thiazolebutanol;
2-[[(1*R*,3*S*)-3-amino-4-hydroxy-1-(5-thiazolyl)butyl]thio]-6-(trifluoromethyl)- 3-pyridinecarbonitrile;
2-[[(1*R*,3*S*)-3-amino-4-hydroxy-1-(5-thiazolyl)butyl]thio]-5-chloro-benzonitrile;
and pharmaceutically acceptable salts thereof.

Unless otherwise indicated, the term "C1 to 4 alkyl" referred to herein denotes a straight or branched chain alkyl group having from 1 to 4 carbon atoms. Examples of such groups include methyl, ethyl, n-propyl, i-propyl, n-butyl, i-butyl and t-butyl.

Unless otherwise indicated, the term "C1 to 4 alkoxy" referred to herein denotes a straight or branched chain alkoxy group having from 1 to 4 carbon atoms. Examples of such groups include methoxy, ethoxy, n-propoxy, i-propoxy and t-butoxy.

Unless otherwise indicated, the term "halogen" referred to herein denotes fluoro, chloro, bromo and iodo.

Examples of a "C1 to 4 alkyl or C1 to 4 alkoxy optionally further substituted by one or more fluorine atoms" include CH₂F, CHF₂, CF₃, CF₃CF₂, CF₃CH₂, CH₂FCH₂, CH₃CF₂, CF₃CH₂CH₂, OCF₃ and OCH₂CF₃.

According to the invention, we further provide a process for the preparation of compounds of formula (I), or a pharmaceutically acceptable salt, enantiomer or racemate thereof which comprises:
(a) reaction of a compound of formula (II) 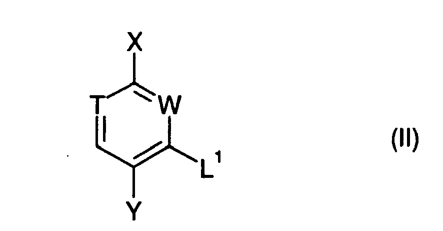 wherein T, X, Y and W are as defined in formula (I) and L¹ represents a leaving group, with a compound of formula (III) 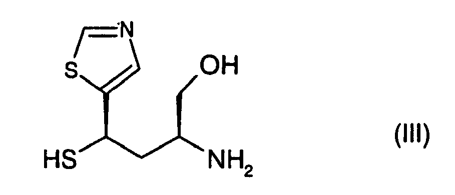 or
(b) reaction of a compound of formula (IV) 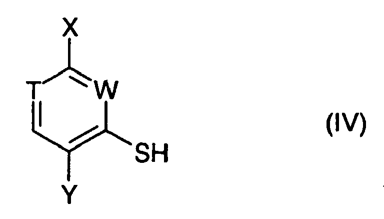 wherein T, W, X and Y are as defined in formula (I), with a compound of formula (V) 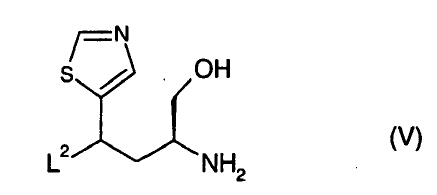 wherein L² is a leaving group;
   and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

In process (a), the reaction is performed by treating a nucleophile of formula (III) with an electrophile of formula (II) in an inert solvent. Suitable leaving groups L¹ include sulphonates and halides, particularly fluoride or chloride. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran and dimethylsulfoxide. The reaction is generally conducted at a temperature between 0 °C and the boiling point of the solvent.

In process (b), the reactants (IV) and (V) are coupled together in a suitable inert solvent such as tetrahydrofuran using, for example, Mitsunobu conditions. Thus, for example, the reactants are treated with a phosphine derivative and an azo derivative at a suitable temperature, generally between 0 °C and the boiling point of the solvent. Suitable phosphine derivatives include triphenylphosphine and tributylphosphine. Suitable azo derivatives include diethyl azodicarboxylate, diisopropyl azodicarboxylate and 1,1'-(azodicarbonyl)dipiperidine. Suitable leaving groups L² include hydroxy.

Alternatively in process (b), the reaction is performed by treating a nucleophile of formula (IV) with an electrophile of formula (V) in an inert solvent. Suitable leaving groups L² include sulphonates and halides, particularly chloride or bromide. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran and dimethylsulfoxide. The reaction is generally conducted at a temperature between 0 °C and the boiling point of the solvent.

It will be apparent to a person skilled in the art that in the above processes it may be desirable or necessary to protect an amine or hydroxyl or other potentially reactive group. Suitable protecting groups and details of processes for adding and removing such groups may be found by reference to the standard text "Protective Groups in Organic Synthesis", 3rd Edition (1999) by Greene and Wuts.

In one preferred embodiment, amine groups are protected as carbamate derivatives, for example, as t-butyloxycarbamates.

In another particularly preferred embodiment, the amine and hydroxyl groups of compounds of formula (I) are protected simultaneously as a cyclic hemi-aminal as in formula (VI).

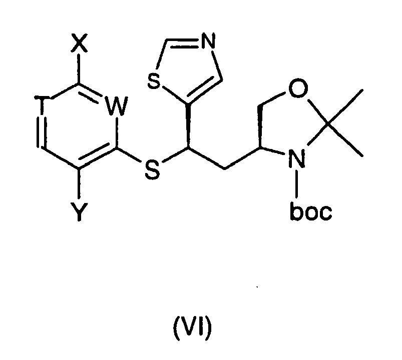

Specific examples of the use of protecting groups are given in the Examples section.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

Salts of compounds of formula (I) may be formed by reacting the free base, or a salt, enantiomer or racemate thereof, with one or more equivalents of the appropriate acid. The reaction may be carried out in a solvent or medium in which the salt is insoluble or in a solvent in which the salt is soluble, for example, water, dioxane, ethanol, tetrahydrofuran or diethyl ether, or a mixture of solvents, which may be removed *in vacuo* or by freeze drying. The reaction may also be a metathetical process or it may be carried out on an ion exchange resin.

Intermediate compounds may be used as such or in protected form. Protecting groups and details of processes for their removal may be found by reference to the standard text "Protective Groups in Organic Synthesis", 3rd Edition (1999) by Greene and Wuts.

The compounds of the invention and intermediates thereto may be isolated from their reaction mixtures and, if necessary further purified, by using standard techniques.

The compounds of formula I may exist in enantiomeric forms. Therefore, all enantiomers, diastereomers, racemates and mixtures thereof are included within the scope of the invention. The various optical isomers may be isolated by separation of a racemic mixture of the compounds using conventional techniques, for example, fractional crystallisation, or HPLC.

Intermediate compounds may also exist in enantiomeric forms and may be used as purified enantiomers, diastereomers, racemates or mixtures.

The compounds of formula (I) and their pharmaceutically acceptable salts are useful because they possess pharmacological activity in animals. In particular, the compounds are active as inhibitors of the enzyme nitric oxide synthase. More particularly, they are inhibitors of the inducible isoform of the enzyme nitric oxide synthase and as such are predicted to be useful in therapy, for example, as anti-inflammatory agents. They may also have utility as inhibitors of the neuronal isoform of the enzyme nitric oxide synthase.

The compounds and their pharmaceutically acceptable salts are indicated for use in the treatment or prophylaxis of diseases or conditions in which synthesis or oversynthesis of nitric oxide synthase forms a contributory part. In particular, the compounds are indicated for use in the treatment of inflammatory conditions in mammals including man.

Conditions that may be specifically mentioned are:
osteoarthritis, rheumatoid arthritis, rheumatoid spondylitis, gouty arthritis and other arthritic conditions, inflamed joints;
eczema, psoriasis, dermatitis or other inflammatory skin conditions such as sunburn; inflammatory eye conditions including uveitis, glaucoma and conjunctivitis;
lung disorders in which inflammation is involved, for example, asthma, bronchitis, chronic obstructive pulmonary disease, pigeon fancier's disease, farmer's lung, acute respiratory distress syndrome;
bacteraemia, endotoxaemia (septic shock), aphthous ulcers, gingivitis, pyresis, pain, meningitis and pancreatitis;
conditions of the gastrointestinal tract including inflammatory bowel disease, Crohn's disease, atrophic gastritis, gastritis varialoforme, ulcerative colitis, coeliac disease, regional ileitis, peptic ulceration, irritable bowel syndrome, reflux oesophagitis, damage to the
gastrointestinal tract resulting from infections by, for example, *Helicobacter pylori,* or from treatments with non-steroidal anti-inflammatory drugs;
and other conditions associated with inflammation.

The compounds will also be useful in the treatment and alleviation of acute pain or persistent inflammatory pain or neuropathic pain or pain of a central origin.

We are particularly interested in the conditions inflammatory bowel disease, rheumatoid arthritis, osteoarthritis, chronic obstructive pulmonary disease and pain.

The compounds of formula (I) and their pharmaceutically acceptable salts may also be useful in the treatment or prophylaxis of diseases or conditions in addition to those mentioned above. For example, the compounds may be useful in the treatment of atherosclerosis, cystic fibrosis, hypotension associated with septic and/or toxic shock, in the treatment of dysfunction of the immune system, as an adjuvant to short-term immunosuppression in organ transplant therapy, in the control of onset of diabetes, in the maintenance of pancreatic function in diabetes, in the treatment of vascular complications associated with diabetes and in co-therapy with cytokines, for example TNF or interleukins.

The compounds of formula (I) may also be useful in the treatment of hypoxia, for example in cases of cardiac arrest and stroke, neurodegenerative disorders including nerve degeneration and/or nerve necrosis in disorders such as ischaemia, hypoxia, hypoglycaemia, epilepsy, and in external wounds (such as spinal cord and head injury), hyperbaric oxygen convulsions and toxicity, dementia, for example pre-senile dementia, Alzheimer's disease and AIDS-related dementia, Sydenham's chorea, Parkinson's disease, Tourette's syndrome, Huntington's disease, amyotrophic lateral sclerosis, multiple sclerosis, muscular dystrophy, Korsakoffs disease, imbecility relating to a cerebral vessel disorder, sleeping disorders, schizophrenia, depression, pain, autism, seasonal affective disorder, jet-lag, depression or other symptoms associated with premenstrual syndrome (PMS), anxiety and septic shock. Compounds of formula (I) may also be expected to show activity in the prevention and reversal of drug addiction or tolerance such as tolerance to opiates and diazepines, treatment of drug addiction, treatment of migraine and other vascular headaches, neurogenic inflammation, in the treatment of gastrointestinal motility disorders, cancer and in the induction of labour.

We are particularly interested in the conditions stroke, Alzheimer's disease, Parkinson's disease, multiple sclerosis, schizophrenia, migraine, cancer, septic shock and pain.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dosage administered will, of course, vary with the compound employed, the mode of administration and the treatment desired. However, in general, satisfactory results are obtained when the compounds are administered at a dosage of the solid form of between 1 mg and 2000 mg per day.

The compounds of formula (I), and pharmaceutically acceptable derivatives thereof, may be used on their own, or in the form of appropriate pharmaceutical compositions in which the compound or derivative is in admixture with a pharmaceutically acceptable adjuvant, diluent or carrier. Administration may be by, but is not limited to, enteral (including oral, sublingual or rectal), intranasal, inhalation, intravenous, topical or other parenteral routes. Conventional procedures for the selection and preparation of suitable pharmaceutical formulations are described in, for example, "Pharmaceuticals - The Science of Dosage Form Designs", M. E. Aulton, Churchill Livingstone, 1988. The pharmaceutical composition preferably comprises less than 80% and more preferably less than 50% of a compound of formula (I), or a pharmaceutically acceptable salt thereof.

According to the invention, we further provide a pharmaceutical composition comprising a compound of formula (I), or a pharmaceutically acceptable salt thereof, in admixture with a pharmaceutically acceptable adjuvant, diluent or carrier.

There is also provided a process for the preparation of such a pharmaceutical composition which comprises mixing the ingredients.

The compounds of formula (I), and pharmaceutically acceptable derivatives thereof, may also be advantageously used in combination with a COX inhibitor, more particularly in combination with a COX-2 inhibitor. Particularly preferred COX-2 inhibitors are Celecoxib and MK-966. The NOS inhibitor and the COX-2 inhibitor may either be formulated together within the same pharmaceutical composition for administration in a single dosage unit, or each component may be individually formulated such that separate dosages may be administered either simultaneously or sequentially.

The invention is illustrated, but in no way limited, by the following examples:

The following abbreviations are used:- DMSO (dimethylsulfoxide), DMF (*N,N-*dimethylformamide), THF (tetrahydrofuran), NMP (*N*-methylpyrrolidinone).

### Example 1

### 2-[[(1R,3S)-3-Amino-4-hydroxy-1-(5-thiazolyl)butyl]thio]-5-chloro-3-pyridinecarbonitrile ethanedioate

### a) (4S)-4-[(2R)-2-Hydroxy-2-(2-chloro-5-thiazolyl)ethyl]-2,2-dimethyl-3-oxazolidinecarboxylic acid, 1,1-dimethylethyl ester and (4S)-4-[(2S)-2-hydroxy-2-(2-chloro-5-thiazolyl)ethyl]-2,2-dimethyl-3-oxazolidinecarboxylic acid, 1,1-dimethlyethyl ester

*n*-Butyl lithium (2.0M in hexanes, 21.6 ml) was added dropwise to a solution of 2-chlorothiazole (5.4 g) in THF (400 ml) at -78 °C under a nitrogen atmosphere. After 15 minutes a solution of (4*S*)-2,2-dimethyl-4-(2-oxoethyl)-3-oxazolidinecarboxylic acid, 1,1-dimethylethyl ester (7.0 g) in THF (140 ml) was added dropwise and the reaction mixture was stirred for a further 60 minutes. The cooling was then removed and the mixture was stirred at room temperature for 60 minutes. The reaction mixture was poured into saturated aqueous ammonium chloride and the products extracted with diethyl ether. The combined extracts were washed with brine, dried (magnesium sulfate), filtered and evaporated. Purification by chromatography (silica, 20% ethyl acetate/isohexane as eluent) gave the (4*S*, 2*S*) sub-title compound (5.2 g) as a colourless oil.
MS APCI +ve ^{m}/z 363 [(M+H]⁺).
¹H NMR 400MHz (DMSO-d₆, 90°C) 7.49 (1H, s), 5.69 (1H, d), 4.85 (1H, dd), 3.96-3.86 (2H, m), 3.84-3.77 (1H, m), 2.10-1.99 (1H, m), 1.90 (1H, dt), 1.47 (3H, s), 1.40 (12H, s).

Further elution gave the (4*S*, 2*R*) sub-title compound (5.1 g, 42%) as a colourless oil.
MS APCI +ve ^{m}/z 363 ([M+H]⁺).
¹H NMR 400MHz (DMSO-d₆, 90°C) 7.49 (1H, s), 5.70 (1H, d), 4.92 (1H, apparent quin.), 4.05-3.98 (1H, m), 3.91-3.84 (2H, m), 2.16-2.07 (1H, m), 1.84 (1H, ddd), 1.48 (3H, s),1.41 (12H, s).

### b) (4S)-4-[(2S)-2-Hydroxy-2-(5-thiazolyl)ethyl]-2,2-dimethyl-3-oxazolidinecarboxylic acid, 1,1-dimethylethyl ester

10% Palladium on charcoal was added to a solution of the (4S, 2S) product from Example 1 step (a) (5.2 g) and sodium acetate (2.9 g) in methanol (120 ml). The reaction mixture was stirred at 50 °C under 5 atmospheres of hydrogen for 16 h. The mixture was filtered and evaporated. The residue was then dissolved in dichloromethane, re-filtered and evaporated to give the sub-title compound (4.8 g) as a colourless oil.
MS APCI +ve ^{m}/z 329 ([M+H]⁺).
¹H NMR 400MHz (DMSO-d₆, 90°C) 8.90 (1H, d), 7.73 (1H, d), 5.49 (1H, d), 4.94 (1H, dt), 3.93-3.91 (2H, m), 3.85-3.78 (1H, m), 2.09 (1H, ddd), 1.94-1.89 (1H, m), 1.47 (3H, s), 1.41 (3H, s), 1.40 (9H, s).

### c) (4S)-4-[(2R)-2-(Benzoylthio)-2-(5-thiazolyl)ethyl]-2,2-dimethyl-3-oxazolidinecarboxylic acid, 1,1-dimethylethyl ester

To a solution of triphenylphosphine (7.9 g) in dry THF (100 ml) at 0 °C was added diisopropylazodicarboxylate (5.9 ml) dropwise over 5 minutes. After the addition was complete the mixture was stirred for 20 minutes and then a solution of the product from Example 1 step (b) (4.8 g) and thiobenzoic acid (3.5 ml) in THF (50 ml) was added. The cooling bath was removed and stirring continued at room temperature overnight. The mixture was concentrated and the residue was purified by chromatography (silica, 10% diethyl ether/*iso*hexane as eluent) to give the sub-title compound (4.4 g) as a yellow solid.
MS APCI +ve ^{m}/z 449 ([M+H]⁺).

### d) (4S)-4-[(2R)-2-[(5-chloro-3-cyano-2-pyridinyl)thio]-2-(5-thiazolyl)ethyl]-2,2-dimethyl-3-oxazolidinecarboxylic acid, 1,1-dimethylethyl ester

The product from Example 1 step (c) (290 mg) was treated with 7M ammonia in methanol (5 ml) and stirred at room temperature for 6 h. The solvent was evaporated, the residue dissolved in dry DMF (5 ml) and treated with 2,5-dichloro-3-pyridinecarbonitrile (112 mg) followed by cesium carbonate (210 mg) under nitrogen. The reaction mixture was stirred for 18 h, poured into brine and ethyl acetate and the organic layer separated, washed with water (2 x), brine and dried (magnesium sulfate). The solvent was evaporated and the residue purified by chromatography (silica, 25% ethyl acetate/dichloromethane as eluent) to give the sub-title compound (160 mg) as a viscous oil.
MS APCI +ve ^{m}/z 481 ([M+H]⁺).

### e) 2-[[(1R,3S)-3-Amino-4-hydroxy-1-(5-thiazolyl)butyl]thio]-5-chloro-3-pyridinecarbonitrile ethanedioate

The product from Example 1 step (d) (160 mg) was stirred in 4M HCl in dioxane (1 ml) and methanol (1 ml) for 1 h. The reaction mixture was evaporated, azeotroped with diethyl ether (3 x), then treated with 1 equivalent of oxalic acid in ethanol (10 ml). The clear solution was treated with diethyl ether until complete precipitation and the solid collected by filtration, washed with diethyl ether and dried *in vacuo* at 40 °C for 2 h to give the title compound (60 mg) as a light brown solid.
MS APCI + ve ^{m}/z 341 ([M+H]⁺).
¹H NMR 400MHz (DMSO-d₆) 9.07 (1H, s), 8.87 (1H, d), 8.56 (1H, d), 8.07 (2H, bs), 8.00 (1H, s), 5.71 (1H, t), 3.57 (1H, dd), 3.49 (1H, dd), 3.12-3.04 (1H, m), 2.45-2.32 (2H, m).

### Example 2

### 2-[[(1R,3S)-3-Amino-4-hydroxy-1-(5-thiazolyl)butyl]thio]-4-chloro-benzonitrile dihydrochloride

### a) (4S)-4-[(2R)-2-[(5-Chloro-2-cyanophenyl)thio]-2-(5-thiazolyl)ethyl]-2,2-dimethyl-3-oxazolidinecarboxylic acid, 1,1-dimethylethyl ester

The product from Example 1 step (c) (290 mg) was treated with 2-fluoro-4-chlorobenzonitrile (101 mg) and cesium carbonate (211 mg) according to the procedure of Example 1 step (d) to give the sub-title compound (160 mg) as a viscous oil.
MS APCI +ve ^{m}/z 480 ([M+H]⁺).

### b) 2-[[(1R,3S)-3-Amino-4-hydroxy-1-(5-thiazolyl)butyl]thio]-4-chloro-benzonitrile dihydrochloride

The product of Example 2 step (a) was stirred in 4M HCl in dioxane (1 ml) and methanol (1 ml) for 1 h. The reaction mixture was evaporated, azeotroped with diethyl ether (3 x) and dried *in vacuo* at 40 °C for 2 h to give the title compound (113 mg) as a pale yellow solid.
MS APCI + ve ^{m}/z 340 ([M+H]⁺).
¹H NMR 400MHz (DMSO-d₆) 9.05 (1H, s), 8.15 (2H, bs), 7.86 (1H, d), 7.83 (1H, s), 7.81 (1H, d), 7.55 (1H, dd), 5.57 (1H, t), 3.59 (1H, dd), 3.51 (1H, dd), 3.07 (1H, br s), 2.31-2.23 (2H, m).

### Example 3

### (2S,4R)-2-Amino-4-[[2-chloro-5-(trifluoromethyl)phenyl]thio]-5-thiazolebutanol hydrochloride

### a) (4S)-4-[(2R)-2-[[2-Chloro-5-(trifluoromethyl)phenyl]thio]-2-(5-thiazolyl)ethyl]-2,2-dimethyl-3-oxazolidinecarboxylic acid, 1,1-dimethylethyl ester

The product from Example 1 step (c) (190 mg) was treated with 4-chloro-3-fluorobenzotrifluoride (83 mg) and cesium carbonate (163 mg) according to the procedure of Example 1 step (d) to give the sub-title compound (105 mg) as a viscous oil.
MS APCI +ve ^{m}/z 523 ([M+H]⁺).

### b) (2S,4R)-2-Amino-4-[[2-chloro-5-(trifluoromethyl)phenyl]thio]-5-thiazolebutanol hydrochloride

The product of Example 3 step (a) was reacted according to the procedure of Example 2 step (b) to give the title compound (58 mg) as a white solid containing the (2S, 4S) diastereomer as a 30% impurity.
MS APCI + ve ^{m}/z 383 ([M+H]⁺).
¹H NMR 300MHz (DMSO-d₆) 9.04 (1H, s), 8.15 (2H, br s), 7.86 (1H, d), 7.77-7.69 (2H, m), 7.61 (1H, s), 5.54 (1H, t), 3.63-3.55 (1H, m), 3.13-3.01 (2H, m), 2.34-2.19 (2H, m).

### Example 4

### 2-[[(1R,3S)-3-Amino-4-hydroxy-1-(5-thiazolyl)butyl]thio]-6-(trifluoromethyl)- 3-pyridinecarbonitrile dihydrochloride

### a) (4S)-4-[(2R)-2-[[3-Cyano-6-(trifluoromethyl)-2-pyridinyl]thio]-2-(5-thiazolyl)ethyl]-2,2-dimethyl-3-oxazolidinecarboxylic acid, 1,1-dimethylethyl ester

The product from Example 1 step (c) (190 mg) was treated with 2-chloro-6-trifluoromethyl-3-pyridinecarbonitrile (86 mg) and cesium carbonate (163 mg) according to the procedure of Example 1 step (d) to give the sub-title compound (117 mg) as a viscous oil.
MS APCI +ve ^{m}/z (515 [M+H]⁺).

### b) 2-[[(1R,3S)-3-Amino-4-hydroxy-1-(5-thiazolyl)butyl]thio]-6-(trifluoromethyl)-3-pyridinecarbonitrile dihydrochloride

The product of Example 4 step (a) was reacted according to the procedure of Example 2 step (b) to give the title compound (85 mg) as a white solid.
MS APCI + ve ^{m}/z (375 [M+H]⁺).
¹H NMR 300MHz (DMSO-d₆) 9.04 (1H, s), 8.57 (1H, d), 8.20 (2H, bs), 7.97 (1H, s), 7.89 (1H, d), 5.68 (1H, t), 3.60-3.56 (1H, m), 3.52 (1H, dd), 3.18-3.09 (1H, m), 2.48-2.41 (2H, m).

### Example 5

### 2-[[(1R,3S)-3-Amino-4-hydroxy-1-(5-thiazolyl)butyl]thio]-5-chloro-benzonitrile dihydrochloride

### a) (4S)-4-[(2R)-2-[(4-Chloro-2-cyanophenyl)thio]-2-(5-thiazolyl)ethyl]-2,2-dimethyl-3-oxazolidinecarboxylic acid, 1,1-dimethylethyl ester

The product from Example 1 step (c) (190 mg) was treated with 5-chloro-2-fluorobenzonitrile (65 mg) and cesium carbonate (163 mg) according to the procedure of Example 1 step (d) to give the sub-title compound (160 mg) as a viscous oil.
MS APCI +ve ^{m}/z 480 ([M+H]⁺).

### b) 2-[[(1R,3S)-3-Amino-4-hydroxy-1-(5-thiazolyl)butyl]thio]-5-chloro-benzonitrile dihydrochloride

The product of Example 5 step (a) was reacted according to the procedure of Example 2 step (b) to give the title compound (97 mg) as a pale yellow solid.
MS APCI + ve ^{m}/z 340 ([M+H]⁺).
¹H NMR 300MHz (DMSO d₆) 9.04 (1H, s), 8.27 (2H, br s), 8.04 (1H, d), 7.81 (1H, s), 7.77-7.74 (2H, m), 5.58 (1H, t), 3.57 (1H, dd), 3.51 (1H, dd), 3.07-3.00 (1H, m), 2.27 (2H, t).

### Screens

The pharmacological activity of compounds according to the invention was tested in the following screens.

### Screen 1

The activity of compounds of formula (I), or a pharmaceutically acceptable salt thereof, may be screened for nitric oxide synthase inhibiting activity by a procedure based on that of Förstermann *et al*., Eur. J. Pharm., 1992, **225**, 161-165. Nitric oxide synthase converts ³H-L-arginine into ³H-L-citrulline which can be separated by cation exchange chromatography and quantified by liquid scintillation counting.

Enzyme is prepared, after induction, from the cultured murine macrophage cell line J774A-1 (obtained from the laboratories of the Imperial Cancer Research Fund). J774A-1 cells are cultured in Dulbeccos Modified Eagles Medium (DMEM) supplemented with 10% foetal bovine serum, 4 mM L-glutamine and antibiotics (100 units/ml penicillin G, 100 mg/ml streptomycin & 0.25 mg/ml amphotericin B). Cells are routinely grown in 225 cm³ flasks containing 35 ml medium kept at 37 °C and in a humidified atmosphere containing 5% CO₂.

Nitric oxide synthase is produced by cells in response to interferon-g (IFNg) and lipopolysaccharide (LPS). The medium from confluent culture flasks is removed and replaced with 25 ml (per flask) of fresh medium containing 1 mg/ml LPS and 10 units/ml IFNg. After a period of 17-20 hours in culture, harvesting of cells is accomplished by scraping the cell sheet from the flask surface into the culture medium. Cells are collected by centrifugation (1000 g for 10 minutes) and lysate prepared by adding to the cell pellet a solution containing 50 mM Tris-HCl (pH 7.5 at 20 °C), 10% (v/v) glycerol, 0.1 % (v/v) Triton-X-100, 0.1 mM dithiothreitol and a cocktail of protease inhibitors comprising leupeptin (2 mg/ml), soya bean trypsin inhibitor (10 mg/ml), aprotinin (5 mg/ml) and phenylmethylsulphonyl fluoride (50 mg/ml).

For the assay, 25 µl of substrate cocktail (50 mM Tris-HCl (pH 7.5 at 20 °C), 400 µM NADPH, 20 µM flavin adenine dinucleotide, 20 µM flavin mononucleotide, 4 µM tetrahydrobiopterin, 12 µM L-arginine and 0.025 mCi L-[³H] arginine) is added to wells of a 96 well filter plate (0.45µM pore size) containing 25 µl of a solution of test compound in 50 mM Tris-HCl. The reaction is started by adding 50 µl of cell lysate (prepared as above) and after incubation for 1 hour at room temperature is terminated by addition of 50 µl of an aqueous solution of 3 mM nitroarginine and 21 mM EDTA.

Labelled L-citrulline is separated from labelled L-arginine using Dowex AG-50W. 150 µl of a 25% aqueous slurry of Dowex 50W (Na⁺ form) is added to the assay after which the whole is filtered into 96 well plates. 75 µl of filtrate is sampled and added to wells of 96 well plates containing solid scintillant. After allowing the samples to dry the L-citrulline is quantified by scintillation counting.

In a typical experiment basal activity is 300 dpm per 75 µl sample which is increased to 1900 dpm in the reagent controls. Compound activity is expressed as IC₅₀ (the concentration of drug substance which gives 50% enzyme inhibition in the assay) and aminoguanidine, which gives an IC₅₀ (50% inhibitory concentration) of 10 µM, is tested as a standard to verify the procedure. Compounds are tested at a range of concentrations and from the inhibitions obtained IC₅₀ values are calculated. Compounds that inhibit the enzyme by at least 25% at 100 µM are classed as being active and are subjected to at least one retest.

In the above screen, the compounds of Examples 1 to 5 were tested and gave IC₅₀ values of less than 10 µM indicating that they are expected to show useful therapeutic activity.

### Screen 2

Recombinant human NO synthases (iNOS, eNOS & nNOS) were expressed in *E*. *coli* and lysates were prepared in Hepes buffer (pH 7.4) containing co-factors (FAD, FMN, H₄B), protease inhibitors, lysozyme and the detergent, CHAPS. These preparations were used, at suitable dilution, to assess inhibition of the various isoforms. Inhibition of NOS was determined by measuring the formation of L-[³H]citrulline from L-[³H]arginine using an adaptation of the method of Förstermann *et al*.⁹ Enzyme assays were performed in the presence of 3 µM [³H]arginine, 1 mM NADPH and other co-factors required to support NOS activity (FAD, FMN, H₄B, calmodulin, Ca²⁺). Since various NOS inhibitors have been reported to exhibit slow binding kinetics, or to inactivate the enzyme in a time dependent manner, enzyme and inhibitor were pre-incubated for 60 min in the presence of NADPH before addition of arginine to initiate the reaction. Incubations continued for a further 60 min before the assays were quenched and [³H]citrulline separated from unreacted substrate by chromatography on Dowex-50W resin in a 96-well format.

In the above screen, the compounds of Examples 1 to 5 were tested and gave IC₅₀ values of less than 10 µM against the iNOS enzyme indicating that they are expected to show useful therapeutic activity.

### Screen 3

Compounds also show activity against the human form of induced nitric oxide synthase as can be demonstrated in the following assay.

The human colorectal carcinoma cell line, DLD-1 (obtained from the European Collection of Animal Cell Culture - cell line number 90102540) was routinely grown in RPMI 1640 supplemented with 10%(v/v) foetal bovine serum, and 2mM L-glutamine, at 37 °C in 5% CO₂.

Nitric oxide synthase was induced in cells by addition of medium containing human recombinant gamma-IFN (1000 units/ml), TNF-alpha (200 U/ml), IL-6 (200 U/ml) and IL-1-beta (250 U/ml). After incubation for 18 hours at 37 °C, the medium was removed and the cells washed with warm phosphate buffered saline. Cells were incubated for a further 5 hours at 37 °C / 5% CO₂ in RPMI 1640 containing 100µM L-arginine and 100µM verapamil-HCl in the presence and absence of test compounds.

Nitrite accumulation was determined by mixing an equal volume of culture media with Griess reagent (10 mg/ml sulphanilamide, 1 mg N-(1-naphthyl)ethylenediamine in 1 ml 2.5% (v/v) phosphoric acid). Inhibition in the presence of compounds was calculated relative to the nitrite levels produced by untreated cells. IC₅₀ values were estimated from a semi-log plot of % inhibition versus concentration of compound.

In this screen the compounds of Examples 1 to 5 gave IC₅₀ values of less than 100 µM, indicating that they are predicted to show useful therapeutic activity.

## Claims

1. A compound of formula (I) 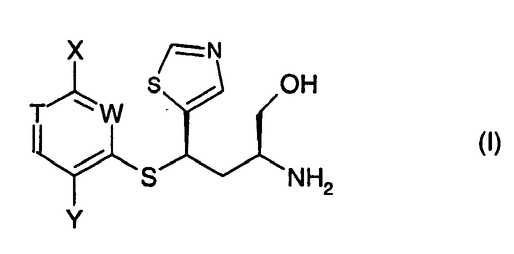 wherein:
T and W independently represent CR¹ or N; and when more than one R¹ group is present, each may be selected independently;
X and R¹ independently represent H, C 1 to 4 alkyl, C 1 to 4 alkoxy, halogen, CN, C≡CH, NO₂, CHO, COCH₃ or NHCHO; said alkyl or alkoxy group being optionally further substituted by one or more fluorine atoms;
Y represents C 1 to 4 alkyl, C 1 to 4 alkoxy, halogen, CN, C≡CH, NO₂, CHO, COCH₃ or NHCHO; said alkyl or alkoxy group being optionally further substituted by one or more fluorine atoms;
or a pharmaceutically acceptable salt thereof.

2. A compound according to Claim 1 wherein Y represents CN or halogen.

3. A compound according to Claim 1 or 2 wherein X and R¹ independently represent H, halogen or CF₃.

4. A compound of formula (I), according to Claim 1, which is:
2-[[(1*R*,3*S*)-3-amino-4-hydroxy-1-(5-thiazolyl)butyl]thio]-5-chloro-3-pyridinecarbonitrile;
2-[[(1*R*,3*S*)-3-amino-4-hydroxy-1-(5-thiazolyl)butyl]thio]-4-chloro-benzonitrile;
(2*S*,4*R*)-2-amino-4-[[2-chloro-5-(trifluoromethyl)phenyl]thio]-5-thiazolebutanol;
2-[[(1*R*,3*S*)-3-amino-4-hydroxy-1-(5-thiazolyl)butyl]thio]-6-(trifluoromethyl)- 3-pyridinecarbonitrile;
2-[[(1*R*,3*S*)-3-amino-4-hydroxy-1-(5-thiazolyl)butyl]thio]-5-chloro-benzonitrile; or a pharmaceutically acceptable salt thereof.

5. A compound of formula (I), according to any one of Claims 1 to 4, or a pharmaceutically acceptable salt thereof, for use as a medicament.

6. A pharmaceutical composition comprising a compound of formula (I) according to any one of Claims 1 to 4, or a pharmaceutically acceptable salt thereof, in admixture with a pharmaceutically acceptable adjuvant, diluent or carrier.

7. The use of a compound of formula (I) according to any one of Claims 1 to 4, or a pharmaceutically acceptable salt thereof, in the manufacture of a medicament for the treatment or prophylaxis of human diseases or conditions in which inhibition of nitric oxide synthase activity is beneficial.

8. The use as claimed in Claim 7 wherein it is predominantly inducible nitric oxide synthase that is inhibited.

9. The use of a compound of formula (I) as defined in any one of Claims 1 to 4, or a pharmaceutically acceptable salt thereof, in the manufacture of a medicament, for the treatment or prophylaxis of inflammatory diseases.

10. The use as claimed in Claim 9 wherein the disease is inflammatory bowel disease.

11. The use as claimed in Claim 9 wherein the disease is rheumatoid arthritis.

12. The use as claimed in Claim 9 wherein the disease is osteoarthritis.

13. The use of a compound of formula (I) as defined in any one of Claims 1 to 4, or a pharmaceutically acceptable salt thereof, in the manufacture of a medicament, for the treatment or prophylaxis of pain.

14. The use of a compound of formula (I) as defined in any one of Claims 1 to 4, or a pharmaceutically acceptable salt thereof, in combination with a COX-2 inhibitor, in the manufacture of a medicament, for the treatment or prophylaxis of inflammatory diseases.

15. A process for the preparation of a compound of formula (I), as defined in any one of Claims 1 to 4, or a pharmaceutically acceptable salt, enantiomer or racemate thereof, wherein the process comprises:
(a) reaction of a compound of formula (II) 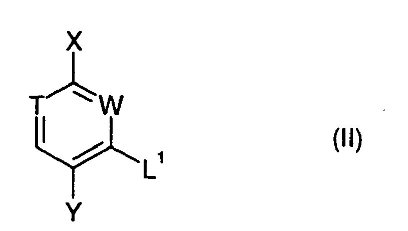 wherein T, X, Y and W are as defined in Claim 1 and L¹ represents a leaving group, with a compound of formula (III) 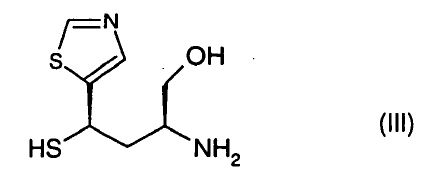 or
(b) reaction of a compound of formula (IV) 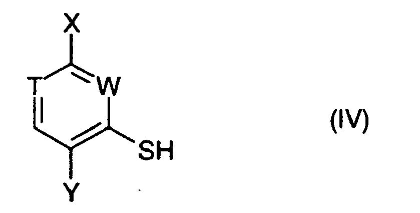 wherein T, W, X and Y are as defined in Claim 1, with a compound of formula (V) 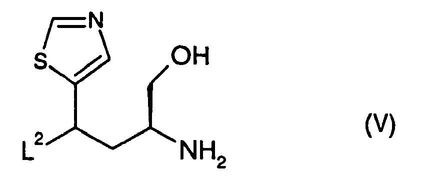 wherein L² is a leaving group;
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Patentansprüche

1. Verbindungen der Formel (I) 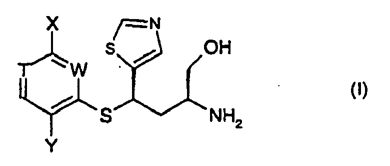 wobei:
T und W unabhängig voneinander für CR¹ oder N stehen und, wenn mehr als eine R¹-Gruppe vorhanden ist, diese jeweils unabhängig voneinander ausgewählt sein können;
X und R¹ unabhängig voneinander für H, C₁₋₄-Alkyl, C₁₋₄-Alkoxy, Halogen, CN, C≡CH, NO₂, CHO, COCH₃ oder NHCHO stehen; wobei die Alkyl- bzw. Alkoxygruppe gegebenenfalls weiter durch ein oder mehrere Fluoratome substituiert ist;
Y für C₁₋₄-Alkyl, C₁₋₄-Alkoxy, Halogen, CN, C≡CH, NO₂, CHO, COCH₃ oder NHCHO steht; wobei die Alkyl- bzw. Alkoxygruppe gegebenenfalls weiter durch ein oder mehrere Fluoratome substituiert ist;
und deren pharmazeutisch annehmbare Salze.

2. Verbindungen nach Anspruch 1, wobei Y für CN oder Halogen steht.

3. Verbindungen nach Anspruch 1 oder 2, wobei X und R¹ unabhängig voneinander für H, Halogen oder CF₃ stehen.

4. Verbindungen der Formel (I) nach Anspruch 1, bei denen es sich um:
2-[[(1*R*,3*S*)-3-Amino-4-hydroxy-1-(5-thiazolyl)butyl]thio]-5-chlor-3-pyridincarbonitril;
2-[[(1*R*,3*S*)-3-Amino-4-hydroxy-1-(5-thiazolyl)butyl]thio]-4-chlorbenzonitril;
(2*S*,4*R*,)-2-Amino-4-[[2-chlor-5-(trifluormethyl)phenyl]thio]-5-thiazolbutanol;
2-[[(1*R*,3*S*)-3-Amino-4-hydroxy-1-(5-thiazolyl)butyl]thio]-6-(trifluormethyl)-3-pyridincarbonitril;
2-[[(1*R*,3*S*)-3-Amino-4-hydroxy-1-(5-thiazolyl)butyl]thio]-5-chlorbenzonitril; handelt
und deren pharmazeutisch annehmbare Salze.

5. Verbindungen der Formel (I) nach einem der Ansprüche 1 bis 4 und deren pharmazeutisch annehmbare Salze zur Verwendung als Medikament.

6. Pharmazeutische Zusammensetzung, enthaltend eine Verbindung der Formel (I) nach einem der Ansprüche 1 bis 4 oder ein pharmazeutisch annehmbares Salz davon in einer Mischung mit einem pharmazeutisch annehmbaren Adjuvants, Verdünnungsmittel oder Träger.

7. Verwendung einer Verbindung der Formel (I) nach einem der Ansprüche 1 bis 4 oder eines pharmazeutisch annehmbaren Salzes davon bei der Herstellung eines Medikaments zur Behandlung oder Prophylaxe von humanen Krankheiten bzw. Leiden, bei denen eine Inhibierung der Stickoxidsynthaseaktivität von Nutzen ist.

8. Verwendung nach Anspruch 7, bei der vorwiegend die induzierbare Stickoxidsynthase inhibiert wird.

9. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von entzündlichen Krankheiten.

10. Verwendung nach Anspruch 9, wobei es sich bei der Krankheit um entzündliche Darmerkrankung handelt.

11. Verwendung nach Anspruch 9, wobei es sich bei der Krankheit um rheumatoide Arthritis handelt.

12. Verwendung nach Anspruch 9, wobei es sich bei der Krankheit um Osteoarthritis handelt.

13. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von Schmerzen.

14. Verwendung einer wie in einem der Ansprüche 1 bis 4 definierten Verbindung der Formel (I) oder eines pharmazeutisch annehmbaren Salzes davon in Kombination mit einem COX-2-Inhibitor bei der Herstellung eines Medikaments zur Behandlung oder Prophylaxe von entzündlichen Krankheiten.

15. Verfahren zur Herstellung einer wie in einem der Ansprüche 1 bis 4 definierten Verbindung der Formel (I) oder eines pharmazeutisch annehmbaren Salzes, Enantiomers oder Racemats davon, bei dem man:
a) eine Verbindung der Formel (II) 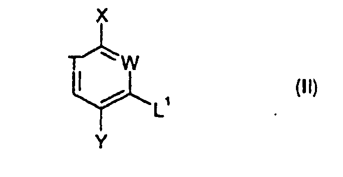 in welcher T, X, Y und W wie in Anspruch 1 definiert sind und L¹ für eine Abgangsgruppe steht, mit einer Verbindung der Formel (III) 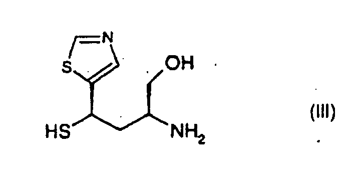 umsetzt oder
b) eine Verbindung der Formel (IV) 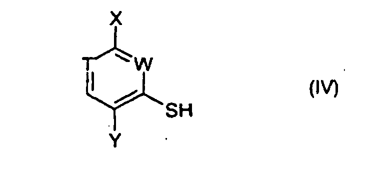 in welcher T, W, X und Y wie in Anspruch 1 definiert sind,
mit einer Verbindung der Formel (V) 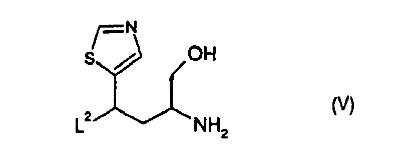 in welcher L² für eine Abgangsgruppe steht, umsetzt;
und, falls gewünscht oder erforderlich, die so erhaltene Verbindung der Formel (I) oder ein anderes Salz davon in ein pharmazeutisch annehmbares Salz davon umwandelt; oder eine Verbindung der Formel (I) in eine andere Verbindung der Formel (I) umwandelt; und, falls gewünscht, die so erhaltene Verbindung der Formel (I) in ein optisches Isomer davon umwandelt.

## Revendications

1. Composé de formule (I) 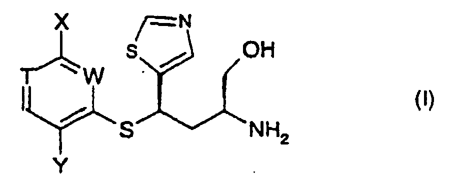 dans laquelle :
T et W représentent indépendamment CR¹ ou N ; et lorsque plus d'un groupement R¹ est présent, chacun peut être choisi indépendamment ;
X et R¹ représentent indépendamment H, alkyle en C1 à 4, alcoxy en C1 à 4, halogène, CN, C≡CH, NO₂, CHO, COCH₃ ou NHCHO ; ledit groupement alkyle ou alcoxy étant éventuellement en outre substitué par un ou plusieurs atomes de fluor ;
Y représente alkyle en C1 à 4, alcoxy en C1 à 4, halogène, CN, C≡CH, NO₂, CHO, COCH₃ ou NHCHO ; ledit groupement alkyle ou alcoxy étant éventuellement en outre substitué par un ou plusieurs atomes de fluor ;
ou un sel pharmaceutiquement acceptable de celui-ci.

2. Composé selon la revendication 1, **caractérisé en ce que** Y représente CN ou halogène.

3. Composé selon la revendication 1 ou 2, **caractérisé en ce que** X et R¹ représentent indépendamment H, halogène ou CF₃.

4. Composé de formule (I), selon la revendication 1, **caractérisé en ce qu'**il s'agit de :
2-[[(1*R*,3*S*)-3-amino-4-hydroxy-1-(5-thiazolyl)-butyl]thio]-5-chloro-3-pyridinecarbonitrile ;
2-[[(1*R*,3*S*)-3-amino-4-hydroxy-1-(5-thiazolyl)-butyl]thio]-4-chlorobenzonitrile ;
(2*S*,4*R*)-2-amino-4-[[2-chloro-5-(trifluorométhyl)-phényl]thio]-5-thiazolebutanol ;
2-[[(1*R*,3*S*)-3-amino-4-hydroxy-1-(5-thiazolyl)-butyl]thio]-6-(trifluorométhyl)-3-pyridine-carbonitrile ;
2-[[(1*R*,3*S*)-3-amino-4-hydroxy-1-(5-thiazolyl)-butyl]thio]-5-chlorobenzonitrile ;
ou un sel pharmaceutiquement acceptable de celui-ci.

5. Composé de formule (I), selon l'une quelconque des revendications 1 à 4, ou un sel pharmaceutiquement acceptable de celui-ci, destiné à être utilisé comme médicament.

6. Composition pharmaceutique comprenant un composé de formule (I) selon l'une quelconque des revendications 1 à 4, ou un sel pharmaceutiquement acceptable de celui-ci, en mélange avec un adjuvant, un diluant ou un support pharmaceutiquement acceptable.

7. Utilisation d'un composé de formule (I) selon l'une quelconque des revendications 1 à 4, ou d'un sel pharmaceutiquement acceptable de celui-ci, dans la fabrication d'un médicament destiné au traitement ou à la prophylaxie de maladies ou d'affections humaines dans lesquelles l'inhibition de l'activité de l'oxyde nitrique synthétase est bénéfique.

8. Utilisation selon la revendication 7, **caractérisée en ce qu'**il s'agit majoritairement de l'oxyde nitrique synthétase inductible qui est inhibée.

9. Utilisation d'un composé de formule (I) tel que défini dans les revendications 1 à 4, ou d'un sel pharmaceutiquement acceptable de celui-ci, dans la fabrication d'un médicament destiné au traitement ou à la prophylaxie de maladies inflammatoires.

10. Utilisation selon la revendication 9, **caractérisée en ce que** la maladie est la maladie inflammatoire de l'intestin.

11. Utilisation selon la revendication 9, **caractérisée en ce que** la maladie est la polyarthrite rhumatoïde.

12. Utilisation selon la revendication 9, **caractérisée en ce que** la maladie est l'ostéoarthrite.

13. Utilisation d'un composé de formule (I) tel que défini dans l'une quelconque des revendications 1 à 4, ou d'un sel pharmaceutiquement acceptable de celui-ci, dans la fabrication d'un médicament destiné au traitement ou à la prophylaxie des douleurs.

14. Utilisation d'un composé de formule (I) tel que défini dans l'une quelconque des revendications 1 à 4, ou d'un sel pharmaceutiquement acceptable de celui-ci, en combinaison avec un inhibiteur de COX-2, dans la fabrication d'un médicament destiné au traitement ou à la prophylaxie de maladies inflammatoires.

15. Procédé de préparation d'un composé de formule (I), tel que défini dans l'une quelconque des revendications 1 à 4, ou d'un sel, d'un énantiomère ou d'un racémate pharmaceutiquement acceptable de celui-ci, **caractérisé en ce que** le procédé comprend :
(a) la réaction d'un composé de formule (II) 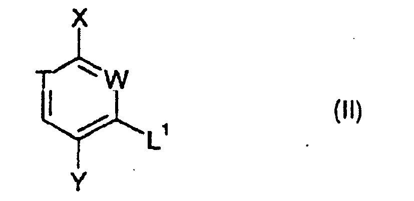 dans laquelle T, X, Y et W sont tels que définis dans la revendication 1 et L¹ représente un groupement partant,
avec un composé de formule (III) 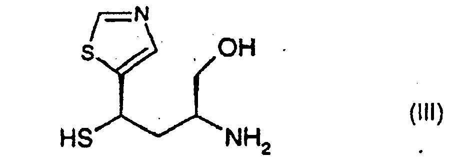 ou
(b) la réaction d'un composé de formule (IV) 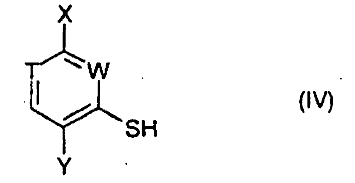 dans laquelle T, W, X et Y sont tels que définis dans la revendication 1,
avec un composé de formule (V) 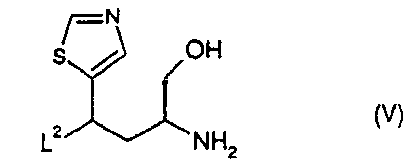 dans laquelle L² est un groupement partant ;
et, si on le souhaite ou s'il est nécessaire, la transformation du composé de formule (I) résultant, ou d'un autre sel de celui-ci, en un sel pharmaceutiquement acceptable de celui-ci ; ou la transformation d'un composé de formule (I) en un autre composé de formule (I) ; et, si on le souhaite, la transformation du composé résultant de formule (I) en un isomère optique de celui-ci.
